# EUROPEAN PATENT APPLICATION

(11) **EP 3 912 650 A1**
(43) Date of publication of application: **24.11.2021**
(21) Application number: 21175410.6
(22) Date of filing: 21.05.2021
(51) Int. Cl.: A61L 9/20, F21S 8/00, F21S 8/04

(54) **AIR AND SURFACE DISINFECTING SYSTEM**

(30) Priority: 23.05.2020 US 202063029463 P; 05.06.2020 US 202063035657 P
(71) Applicant: Medical Illumination International Inc., San Fernando CA 91340 (US)
(72) Inventor: KIVIAT, Alan, California, 91340 (US)
(74) Representative: D Young & Co LLP

(57) **Abstract**

Provided is a disinfecting fixture comprising: a) a body; b) a fan; c) a first irradiation source placed inside of the body, the irradiation source inside of the body configured to disinfect air forced into the body by the fan; d) a conduit for movement of air from outside of the body to inside of the body for irradiation with the first irradiation source, and movement of air outside of the body after irradiation with the first irradiation source; and e) a second irradiation configured to irradiate air outside of the body; wherein air moves with a force generated by the fan from outside of the fixture to inside of the fixture for irradiation with the first irradiation source, and exits the fixture, and wherein air outside of the fixture is irradiated with the second irradiation. Provided is a method for disinfecting light comprising:
a) creating an flow from outside to inside and then outside of a fixture with a fan; b) irradiating the air moved inside of body; c) moving the irradiated air outside with the flow of the air; and d) irradiating air outside of the fixture with a second irradiation source placed outside of the body.

## Description

Disclosed is an ultraviolet light, antimicrobial and antiviral, environmental air and surface disinfecting system and light fixture system.

### Cross-Reference

The present application claims the benefit of US Provisional patent application number. 63/029,463 filed on 05-23-2020, and US provisional patent application number 63/035,657, filed on 06-05-2020, all of which are incorporated by reference here in their entirety.

### Background

A UV-C system combines an ultraviolet germicidal irradiation (UVGI) chamber and air circulating fans. The system uses UV-C light and filtration to draw in and treat environmental air on one side and return air to the room through an exhaust on the other side, reducing microbial and fungal populations in treated air and reduces settling bacteria, viruses and fungi from treated air. A problem with such systems is that there is a need for additional disinfection, which may not be possible with existing systems.

### Detailed Description of the Drawings

Figure 1 shows a bottom view of an embodiment with the light panel open, for example for maintenance or lamp or filter replacement.
Figure 2 shows top section of irradiation chamber.
Figure 3 illustrates a Main body assembly.
Figure 4 illustrates the disinfecting system with a UV light board.
Figure 5 illustrates a wall mounted unit.
Figure 6 illustrates a wall mounted unit.
Figure 7 illustrates various electronic components (plus filter) of the disinfecting system.
Figure 8 illustrates an LED board with an LED source of light.
Figure 9 illustrates placement of UV and or non-UV lights on outside of the body.
Figure 10 illustrates the Fan Enclosure/ Filter Holder.
Figure 11 illustrates a top view of the light panel closed.

### Summary Section of the Invention

The invention is defined in the claims.
Provided in an embodiment is a disinfecting fixture configured to be attached to a ceiling, the fixture comprising: a) a body; b) fan; c) a first irradiation source inside of the body, the irradiation source inside of the body configured to disinfect air forced into the body by the fan; d) a second irradiation source configured to irradiate outside of the body; e) a door attached to the body for accessing inside of the body; f) optionally one or more light sources to provide illumination. The door of any of the above embodiments can be pivotally attached on the long side of the body, which is typically rectangular in shape. In any of the above embodiments, the ends of body, the bottom can have one or more grilles or integrated louvers for movement of air and one or more UV modules (second source). In any of the above embodiments, the grilles or integrated louvers and the UV module can be placed on the bottom of the body at the ends, after where the door ends. In any of the above embodiments, one UV module can be placed at each corner of the body. In any of the above embodiments, the ends of the body can have intake/exhaust for the air, which is forced inside the body with one or more fans. In any of the above embodiments, the fixture comprises an airway/conduit for passage of air from outside, such as through openings/grilles/louvers and/or filters **20,** to the first irradiation source, and passage of the irradiated and/or filtered air to outside of the body. In any of the above embodiments, the light from the UV light inside of the body (first source) is retained inside of the body. In any of the above embodiments, the UV light inside of the body can be placed behand a removable panel that is above the door. The panel has to be opened to access the UV light chamber. In any of the above embodiments, the chamber can have an air baffle on top preventing UV light rays from escaping through the grilles or integrated louvers and creating turbulent flow in the irradiation chamber. In any of the above embodiments, the chamber can have an air riser scoop that directs air into the irradiation chamber and reduces reflection. In any of the above embodiments, one or more light sources to provide illumination can be placed below the door. In any of the above embodiments, the light sources, such as LED or OLED, can be coupled with a suitable diffuser/lens, and/or reflective back plate/paper.

In any of the above embodiments, the disinfecting agent can be a wall mounted fixture, comprising a) a body; b) fan; c) a first irradiation source inside of the body, the irradiation source inside of the body configured to disinfect air forced into the body by the fan; d) an optional second irradiation configured to irradiate outside of the body; e) optionally one or more light sources to provide illumination. In the wall mounted embodiment, the fixture that is mounted on the wall can have a vertical orientation, with horizontal grilles or integrated louvers on the top and/or bottom of the front face of the fixture, a mounting mechanism on the back, and an optional lighting source, such as in a horizontal orientation. The wall mounting unit can lack a pivoting door. The wall mounting unit can have access to the UV chamber inside the unit from the front face of the unit. The wall mounting unit can have any of the embodiments discussed above, including one or more UV lights on the outside, irradiation chamber, diffuser light, UV irradiation modules, air intake grille, exhaust grille, motion sensor, and other optional sensors and electronic components.

### Detailed Description of the Invention

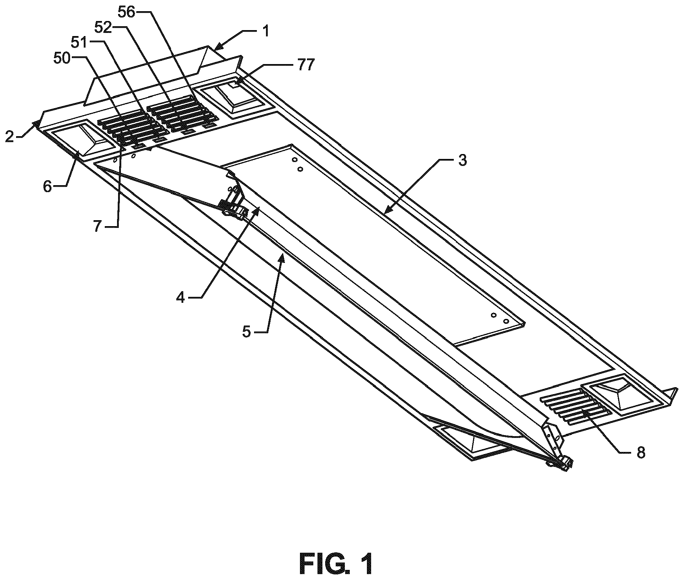

Figure 1 shows a bottom view of an embodiment with the light panel open, for example for maintenance or lamp or filter replacement. Illustrated in figure 1 are UV (Ultra Violet) irradiation chamber **1,** body **2,** UV irradiation chamber access door **3,** main access door **4,** optional downlight **5** (can also be a decorative element without a light source), UV irradiation modules **6** with UV light source **77,** air intake grille **7,** and exhaust grille **8.**

The disinfecting system can have two different embodiments. In one embodiment, the disinfecting system is configured only to disinfect/purify the air in a room. In another embodiment, the system is configured in addition to disinfecting/purifying, to also illuminate a room by having one or more light sources. The one or more light sources can be LED (Light Emitting Diode) or OLED (Organic Light Emitting Diode) light sources **61.**

The disinfecting system can be in form of a fixture with a body **2** that is configured to be attached to a ceiling. The fixture can have a main access door **4** that is pivotally attached to one side of the body **2,** allowing a technician to open the door **4** from the bottom of the fixture and access the electronics inside of the body **2.** In the version with light sources providing a down light, the light sources can be placed directly on a bottom side of the main access door **4.** In the version of the fixture with no downlight, the main access door **4** can be plain or have a decorative panel.

The body **2** of the fixture can have one or more grilles or integrated louvers **(7, 8),** and/or one more irradiation modules 6 that are placed on the sides of the access door **4.** The bottom surface of the grilles or integrated louvers **(7, 8),** and/or one more irradiation modules **6** can be flush with the bottom surface of the body **2** or recessed from the bottom surface of the body **2.** In one embodiment, the grilles or integrated louvers **(7, 8)** are placed at the ends of the body **2** in the center, with the irradiation sources placed at one more or more, or all of the corners of the body **2.** Alternatively, the irradiation sources can be placed along the long edge of the body **2.**

Under the grilles or integrated louvers **(7, 8)** are one or more fans **19** and a filter **20.** The fans **19** bring the air into the unit and into the irradiation chamber. The filter **20** functions to remove large dust particles and to keep the irradiation chamber clean. The exhaust grille **8** directs the clean air out of the unit to circulate within the room. There may be a separate filter on the exhaust grille **8** to catch deactivated pathogens.

The UV modules may contain LEDs, low pressure Mercury lamps, or far UV lamps, which are in the 207 nm or 222 nm range. These lamps can be Krypton-Bromine or Krypton-Chlorine Excimer Lamps. The UV Irradiation modules **6,** in the embodiment are placed on each corner of the system (such as from 0 to 5 inches from the edge of any inlet or exhaust point), and installed on units primarily to provide surface disinfection when the room is not occupied. They may use UV light, with wavelengths anywhere from 200nm to 280nm. The modules may alternatively be provided as two tubular or longer light sources. Alternatively, one or more longer UV light sources may be placed along the edges of the unit. The surface disinfection modules combine with the air purification system to reduce viable pathogens in the area below the system. In another embodiment, UV light having wavelengths of 207 nm or 222 nm may be used, for example, so that the surface infection modules can remain on when the room is occupied. The UV modules allow the system to more effectively disinfect the air in the room before the air has been drawn into the irradiation chamber intake. In another embodiment, UV light of 253.7 nm may be used to provide both surface disinfection and air disinfection. In other embodiments, the modules can operate either continuously or intermittently, based on information from the sensors or from a scheduled cycle. For example, the modules may be programmed to operate when the motion sensors **50** detect that someone has entered the room, or, alternatively, when they detect that someone has entered and left a room. In an alternative embodiment, sound sensors **51** may be provided to detect human activity in the room, and the system is programmed to operate the modules in response to detected sounds. In one example, the system may detect the sound of a person sneezing or coughing, and in response, activate the modules for a period of time to disinfect the air.

A motion sensor **50** can be used if harmful UV rays are used in the irradiation modules to ensure that they are not on when anyone is in the room.

Other sensors can also be used. Another sensor, such as a heat (temperature) sensor **52,** may be used if the unit is installed in patient rooms or ICU (Intensive Care Unit) rooms or similar areas where there are patients who do not move so that the surface disinfection modules do not function.

The fixture can have other electrical components, including, but not limited to, ballast/controller **53,** power supplies (power management unit **57),** a UV light measurement sensor **78,** a safety switch for the UV access door, wiring, and controls for remote operation **58** and indicator **56.** The control system can use a system (which may be wireless **54)** that will be used to operate the functions of the unit (UV air system, UV surface system, down light on/off, down light dimming) where they can be manually turned on or off (various switches **55),** or programmed to be on a schedule. Indicator **56** can also indicate any notifications or errors such as: Filter replacement, UV lamp replacement, UV intensity in the irradiation chamber, fan malfunction, UV module replacement, unit not able to come on as scheduled due to motion sensor activation, unit not able to come on as scheduled due to other (heat) sensor activation, UV chamber door open, and downlight malfunction.

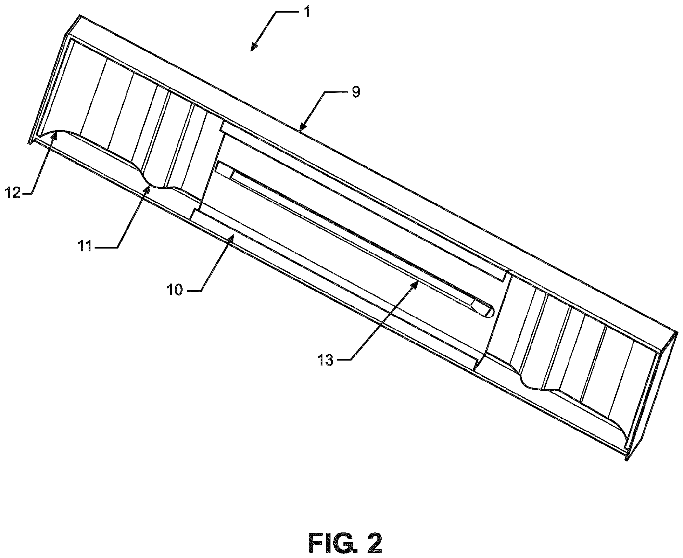

Figure 2 illustrates the top section of irradiation the chamber. Illustrated in this drawing are UV irradiation chamber body **9,** UV irradiation chamber reflector **10,** air baffle top **11,** air riser scoop **12,** and UV light source **13.** The UV irradiation chamber reflector **10** increases UV exposure to the air as it passes through the unit. The air baffle (top) **11** prevents UV light rays from escaping through the grilles or integrated louvers and creates turbulent flow in the irradiation chamber. The air baffle **11** can be curved. The air riser scoop **12** is built into the UV Irradiation chamber body **9** directs air into the irradiation chamber and reduces reflection. In figure 2, the UV light source **13** is shown as a low- pressure mercury lamp, but could also be any UV light source such as LEDs, or Kr-Cl sources.

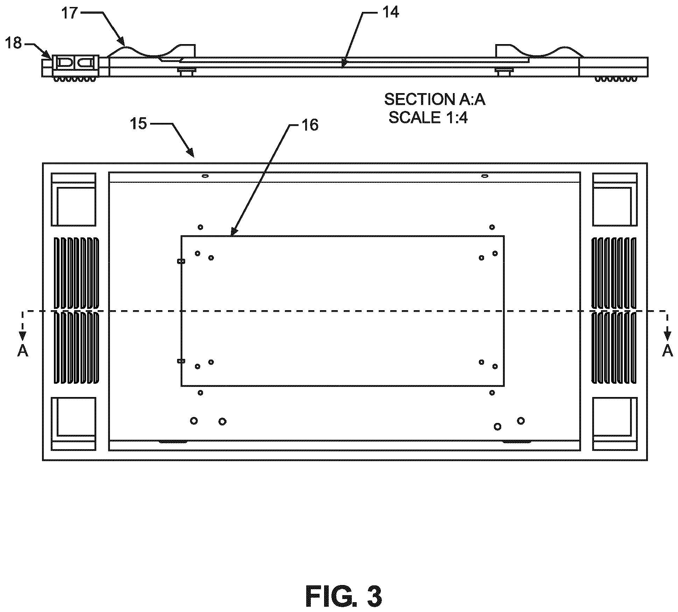

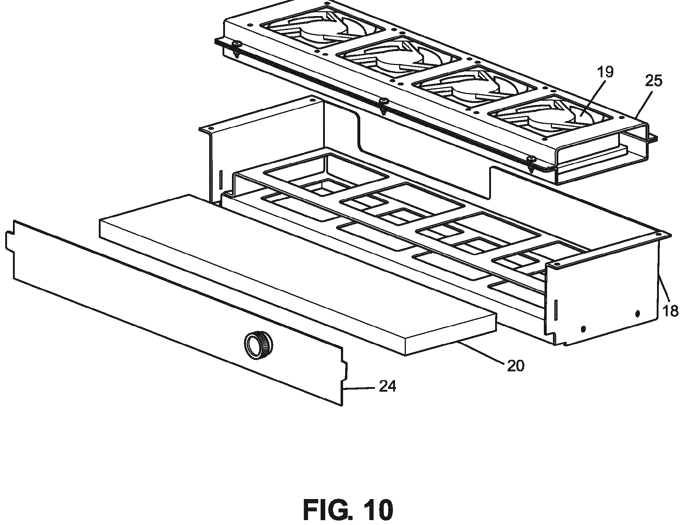

Figure 3 illustrates the body **2** assembly. Illustrated in this figure are main troffer body **14,** front panel for troffer body **15,** access door for UV irradiation chamber **16,** air baffle bottom **17,** and fan tray **18.** The fans **19,** and filter **20** that are placed in fan tray **18** are illustrated in detail in figure **10****.**

The front panel for troffer body **15** can include louvres for the intake and outlet vents. The access door for UV irradiation chamber **16** allows accessing the UV lamp for replacement. There can be a momentary disconnect switch **55** that will turn off UV lamp if the access door is opened. The air baffle bottom **17** can prevent UV light rays from escaping through the grilles or integrated louvers and creates turbulent flow in the irradiation chamber. The fan tray **18** can mount fans **19** in place and holds filter **20** in place. Figure 10 illustrates an exploded view of the fan including the fan **19** and filter **20.** There can be 1-6 fans **19** at one or both ends, such as 4 fans are used in this version. The filter **20** can be a MERV6 filter for removal of large dust particles in order to keep the irradiation chamber clean. There can be one or two fan trays **18.** Typically one fan tray **18** is placed where the air enters the fixture.

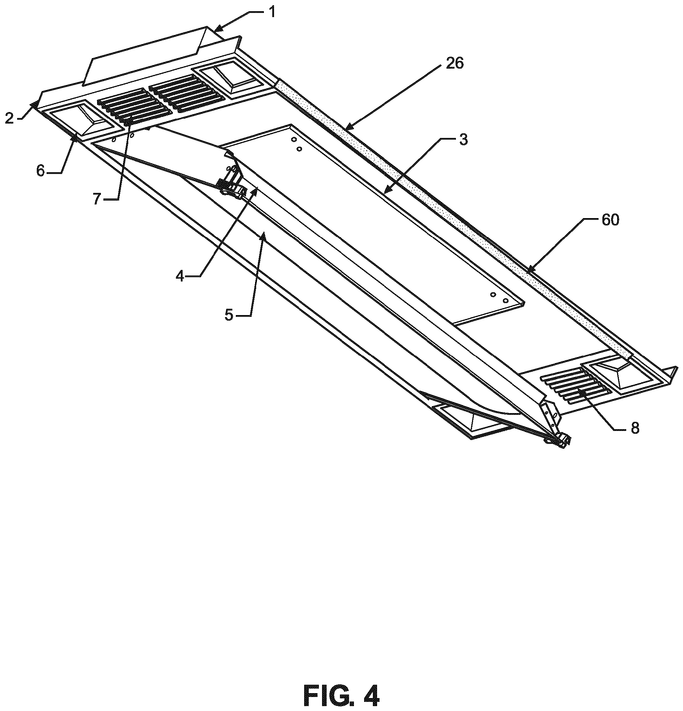

Figure 4 illustrates a UV LED board placed along the length of the body **2.** The LED board **26** can also be present only for the purposes of illumination, not irradiation. In this embodiment, the front access door **4** can be smaller in width to accommodate for placement of the LED board **26** containing LED light sources.

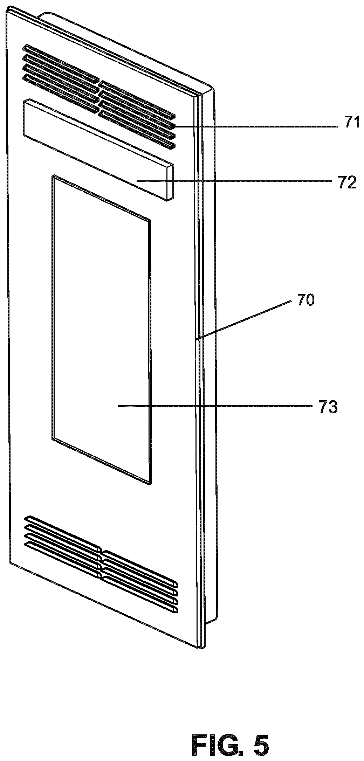

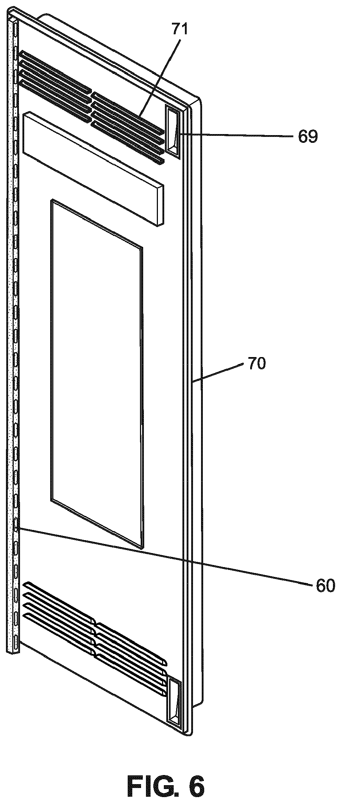

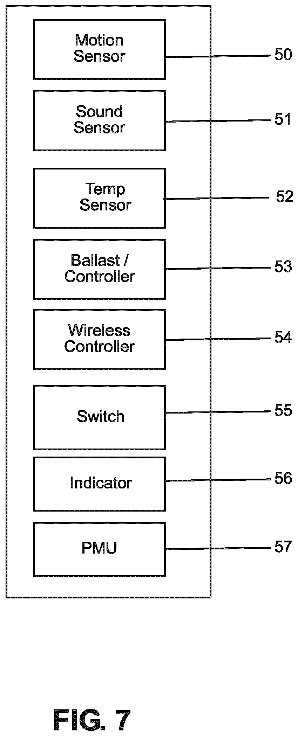

Figures 5 and 6 illustrate a wall mount version of the disinfecting system. The body **70** as illustrated is configured to be mounted on a wall in a vertical fashion, with grilles or integrated louvers **71** on both the top and the bottom of the body **70.** An optional light (for illumination) **72,** placed such as in a horizontal manner, can run parallel and below the front facing grilles or integrated louvers **71** on top of the body. The wall mounted version can have a flat front and a panel **73** that is accessible from the front of the unit. A pivoting door can be absent altogether from front of the wall mounted unit. Figure 7 illustrates placement of LED boards **60** and modules **69,** which can be placed on the front face of the body. The unit can also have UV boards and modules placed on the front face of the body **70.**

Figure 7 illustrates various electronic components of the disinfecting system. Illustrated in this figure is motion sensor **50,** which can sense whether an individual is or is not present in a room, and accordingly switch **55** on or off external UV lights that are not safe for individuals. Sound sensor **51** can also be used to sense the presence of people, and can be used alone or in conjunction with motion sensor **50.** The fixture can have a temperature sensor **52** for determining the temperature of the room. The fixture can have a ballast/controller **53** and/or a power management unit **57,** for managing power to the UV and white lights, sensors, and fans. The fixture can have a wireless controller **54,** such as with Bluetooth, to allow a user to turn on and off the fixture, and/or control the intensity of the fan and/or the light. The fixture can have switch **55,** which can be turned on and off manually, with the wireless controller **54,** or internally if a panel is opened by a technician or the fixture needs maintenance. The fixture can have one or more indicators **56,** which can indicate when the UV light is on in the chamber and/or outside of the chamber, and/or indicate the need for maintenance, such as replacement of the UV light.

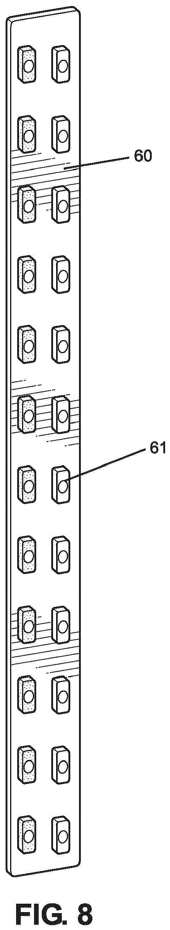

Figure 8 illustrates an LED board **60** with LED sources of light **61.** The LEDs can be placed in one or more rows over the board. The LEDs can be one or different colors, and some instances one set of LEDs producing only UV light while other producing while light for purposes of illumination.

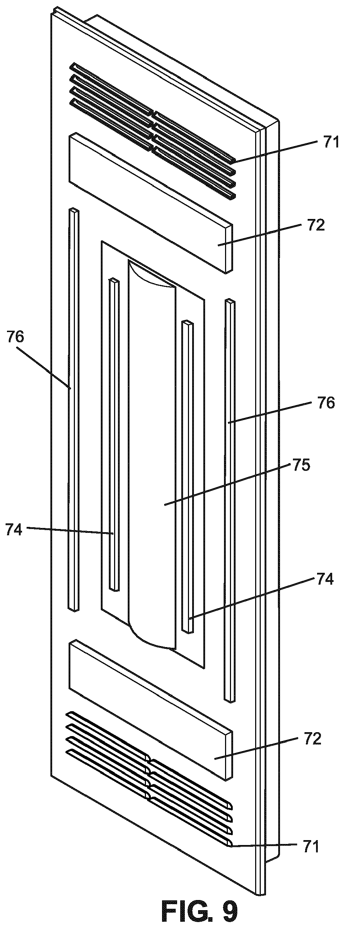

Figure 9 illustrates placement of various UV and/or non-UV lights outside of the body. One or more of the light sources illustrated in figure 10 can be used, such as light with diffuser **72, 74, 75,** and **76.** Visible in this figure is the transparent diffuser for the light source, which is placed inside of the diffuser. The lights can be used in pairs. The lights can be dedicated UV lights for the purpose of disinfection or provide light in the visible spectrum, including white light for the purpose of illumination, and/or provide both visible light and UV disinfecting light.

Figure 10 illustrates exploded view of an assembly with a fan **19** and filter **20.** Illustrated in this view are four fans **19,** which are placed in fan enclosure **25.** Fan Tray **18** holds the fan enclosure **25** and filter **20.** The assembly can have a service panel **24** configured to allow for change of the filter **20** and/or fans **19.**

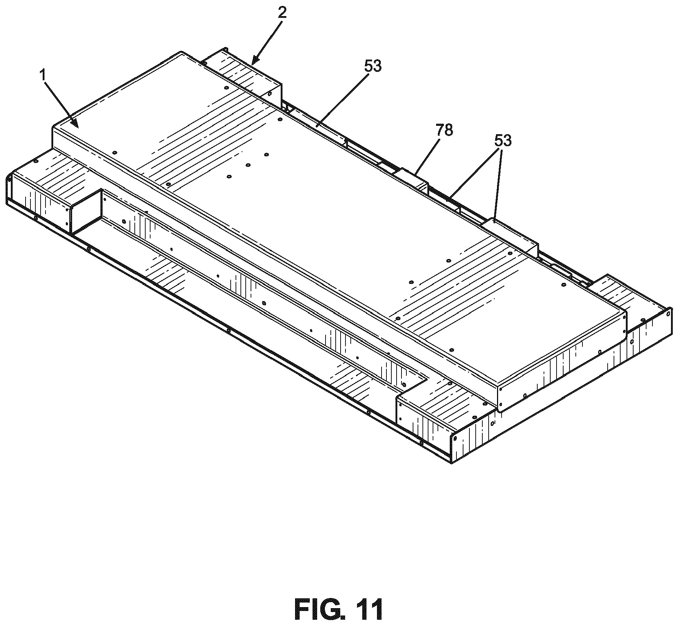

Figure 11 illustrates a top view of the fixture. Illustrated in figure 11 are the UV irradiation chamber **1** and body **2.** Illustrated in this view are ballast and controllers **53.** There can be two or more ballasts and controllers **53.** One set of ballasts and controllers can control the light, another for the fans and sensors, and another for the UV lamp. Additional ballasts and controllers can control the UV modules or lamps for irradiating air and surfaces outside of the fixture. The fixture can also have a UV sensor **78,** which measures the intensity of the UV light in the irradiation chamber and can output the reading to monitor the effective intensity.

Also provided are methods for irradiating air, and disinfecting air with a fixture. The method includes using any fixture as described above to disinfect the air. The method can include sucking air inside of the body of the fixture with one or more fans **19,** irradiating the air inside of the fixture, and pushing the irradiated air out of the fixture with the same or different fans. The method further includes irradiating air outside of the body with an additional irradiation source. The method can further include irradiating air outside first with an outside irradiation source, and then with an inside irradiation source. The method can further include irradiating air inside of the fixture first with an inside irradiation source, and then outside of the fixture with an outside irradiation source. The method can further include irradiating air outside of the fixture, then inside of the fixture, and then outside of the fixture.

Thus, from one perspective, there has now been described a disinfecting fixture comprising: a) a body; b) a fan; c) a first irradiation source placed inside of the body, the irradiation source inside of the body configured to disinfect air forced into the body by the fan; d) a conduit for movement of air from outside of the body to inside of the body for irradiation with the first irradiation source, and movement of air outside of the body after irradiation with the first irradiation source; and e) a second irradiation configured to irradiate air outside of the body; wherein air moves with a force generated by the fan from outside of the fixture to inside of the fixture for irradiation with the first irradiation source, and exits the fixture, and wherein air outside of the fixture is irradiated with the second irradiation. There has also now been described a method for disinfecting light comprising: a) creating an flow from outside to inside and then outside of a fixture with a fan; b) irradiating the air moved inside of body; c) moving the irradiated air outside with the flow of the air; and d) irradiating air outside of the fixture with a second irradiation source placed outside of the body.

### References

- 1.: UV Irradiation Chamber
- 2.: Body
- 3.: UV Irradiation Chamber Access Door
- 4.: Main Access
- 5.: Downlight
- 6.: UV Irradiation modules
- 7.: Air intake grille
- 8.: Exhaust grille
- 9.: UV Irradiation Chamber Body
- 10.: UV Irradiation Chamber Reflector
- 11.: Air Baffle Top
- 12.: Air Riser Scoop
- 13.: UV Light Source
- 14.: Main Troffer Body
- 15.: Front Panel for Troffer Body
- 16.: Access Door for UV Irradiation Chamber
- 17.: Air Baffle Bottom
- 18.: Fan Tray
- 19.: Fans
- 20.: Filter
- 21.: Door/ Reflector Body
- 22.: Reflector
- 23.: Diffuser
- 24.: Service Panel
- 25.: Fan Enclosure
- 26.: Led Board
- 50.: Motion Sensor
- 51.: Sound Sensor
- 52.: Temperature Sensor
- 53.: Ballasts/Controllers
- 54.: Wireless Controller
- 55.: Switch
- 56.: Indicator
- 57.: Power Management Unit (PMU)
- 58.: Wiring and Controls For Remote Operation
- 59.: N/A
- 60.: LED board
- 61.: LED source of light
- 69.: UV-C Module
- 70.: Body/Body
- 71.: Grilles
- 72.: Optional Light
- 73.: Panel
- 74.: Light
- 75.: Light
- 76.: Light
- 77.: UV light source
- 78.: UV sensor

Further examples of feature combinations disclosed herein are set out in the following numbered clauses:
1. A disinfecting fixture comprising:
   a) a body;
   b) a fan;
   c) a first irradiation source placed inside of the body, the irradiation source inside of the body configured to disinfect air forced into the body by the fan;
   d) a conduit for movement of air from outside of the body to inside of the body for irradiation with the first irradiation source, and movement of air outside of the body after irradiation with the first irradiation source; and
   e) a second irradiation configured to irradiate outside of the body;
      wherein air moves with a force generated by the fan from outside of the fixture to inside of the fixture for irradiation with the first irradiation source, and exits the fixture, and wherein the second irradiation source irradiates outside of the fixture.
2. The disinfecting fixture of clause 1, further comprising a door attached to the body for accessing inside of the body.
3. The disinfecting fixture of clause 1 or clause 2, further comprising one or more light sources placed outside of the body to provide illumination with at least a non-UV light wavelength.
4. The disinfecting fixture of any preceding clause, further comprising a door attached to the body, the door comprising one or more sources of light for illumination outside of the fixture.
5. The disinfecting fixture of any preceding clause, further comprising one or more grilles placed on the body for movement of air.
6. The disinfecting fixture of any preceding clause, wherein an edge of the second irradiation source is placed zero to five inches to nearest location where air enters the conduit from outside.
7. The disinfecting fixture of any preceding clause, wherein the second irradiation source irradiates a portion of the air entering the fixture.
8. The disinfecting fixture of any preceding clause, wherein the second irradiation source irradiates a portion of the air leaving the fixture.
9. The disinfecting fixture of any preceding clause, wherein the second irradiation source is in form of a module.
10. The disinfecting fixture of any preceding clause, wherein the second irradiation source is placed in on or more corners of the fixture.
11. The disinfecting fixture of any preceding clause, wherein radiation from the first irradiation source is retained inside of the body.
12. The disinfecting fixture of any preceding clause, wherein the first irradiation source is placed in a chamber behind a removable panel.
13. The disinfecting fixture of clause 12, wherein the chamber has an air baffle that prevents UV light rays from escaping the body.
14. The disinfecting fixture of clause 12 or clause 13, wherein the air baffle is curved.
15. The disinfecting fixture of any of clauses 12 to 14, wherein the chamber has an air riser scoop that directs air into the irradiation chamber.
16. The disinfecting fixture of any preceding clause, wherein the fixture is configured to be mounted on a wall.
17. The disinfecting fixture of any preceding clause, wherein the fixture is configured to be mounted to the ceiling.
18. The disinfecting fixture of any preceding clause, wherein the second irradiation source is 0 to 5 inches from the edge of any inlet or exhaust point where air enters to leaves the fixture.
19. The disinfecting fixture of any preceding clause, further comprising one or more light sources placed outside of the body, the light sources producing differently colored lights.
20. A method for disinfecting air or surfaces comprising:
   a) creating a flow from outside to inside and then outside of a fixture with a fan;
   b) irradiating the air moved inside of body;
   c) moving the irradiated air outside with the flow of the air; and
   d) irradiating air or surfaces outside of the fixture with a second irradiation source placed outside of the body.

## Claims

1. A disinfecting fixture comprising:
a) a body;
b) a fan;
c) a first irradiation source placed inside of the body, the irradiation source inside of the body configured to disinfect air forced into the body by the fan;
d) a conduit for movement of air from outside of the body to inside of the body for irradiation with the first irradiation source, and movement of air outside of the body after irradiation with the first irradiation source; and
e) a second irradiation configured to irradiate outside of the body;
wherein the fan and conduit are configured to move air from outside of the fixture to inside of the fixture for irradiation with the first irradiation source, and to move the air out of the fixture, and wherein air outside of the fixture is irradiated with the second irradiation source.

2. The disinfecting fixture of claim 1, further comprising a door attached to the body for accessing inside of the body.

3. The disinfecting fixture of claim 1 or claim 2, further comprising one or more light sources placed outside of the body to provide illumination with at least a non-UV light wavelength.

4. The disinfecting fixture of any preceding claim, further comprising a door attached to the body, the door comprising one or more sources of light for illumination outside of the fixture.

5. The disinfecting fixture of any preceding claim, further comprising one or more grilles placed on the body for movement of air.

6. The disinfecting fixture of any preceding claim, wherein an edge of the second irradiation source is placed zero to five inches to nearest location where air enters the conduit from outside, and/or wherein the second irradiation source is 0 to 5 inches from the edge of any inlet or exhaust point where air enters to leaves the fixture.

7. The disinfecting fixture of any preceding claim, wherein the second irradiation source is configured to irradiate a portion of the air entering the fixture and/or a portion of the air leaving the fixture.

8. The disinfecting fixture of any preceding claim, wherein the second irradiation source is placed in on or more corners of the fixture.

9. The disinfecting fixture of any preceding claim, wherein the body is configured to retain radiation from the first irradiation source.

10. The disinfecting fixture of any preceding claim, wherein the first irradiation source is placed in a chamber behind a removable panel.

11. The disinfecting fixture of claim 10, wherein the chamber has an air baffle that is configured to prevent UV light rays from escaping the body.

12. The disinfecting fixture of claim 10 or claim 11, wherein the air baffle is curved.

13. The disinfecting fixture of any of claims 10 to 12, wherein the chamber has an air riser scoop that directs air into the irradiation chamber.

14. The disinfecting fixture of any preceding claim, wherein the fixture is configured to be mounted on a wall and/or wherein the fixture is configured to be mounted to the ceiling.

15. A method for disinfecting light comprising:
a) Creating an flow from outside to inside and then outside of a fixture with a fan;
b) Irradiating the air moved inside of body;
c) Moving the irradiated air outside with the flow of the air; and
d) Irradiating air outside of the fixture with a second irradiation source placed outside of the body.
